# EUROPÄISCHE PATENTANMELDUNG

(11) **EP 3 979 256 A1**
(43) Veröffentlichungstag der Anmeldung: **06.04.2022**
(21) Anmeldenummer: 21000258.0
(22) Anmeldetag: 13.09.2021
(51) Int. Cl.: G16H 40/40

(54) **VERFAHREN ZUR AKTUALISIERUNG EINES BEATMUNGSGERÄTS**

(30) Priorität: 02.10.2020 DE 102020006038
(71) Anmelder: Löwenstein Medical Technology S.A., 2557 Luxembourg (LU)
(72) Erfinder: Bychkov, Igor, 76275 Ettlingen (DE); Kopaigorenko, Maria, 76133 Karlsruhe (DE); Schwaibold, Matthias, 76228 Karlsruhe (DE); Born, Benjamin, 76149 Karlsruhe (DE); Eisenreich, Patrick, 76187 Karlsruhe (DE)
(74) Vertreter: Marx, Thomas

(57) **Zusammenfassung**

Verfahren zur Aktualisierung der Firmware und Software eines Beatmungsgerätes (1), wobei die Firmware und die Software des Beatmungsgerätes (1) in einzelne Module (20, 30, 40, 50, 60, 70, 80, 90) unterteilt ist und zu jedem Modul (20, 30, 40, 50, 60, 70, 80, 90) separate Aktualisierungen in Form von Modulaktualisierungen bereitgestellt werden und diese Modulaktualisierungen von zumindest einer räumlich von dem Beatmungsgerät (1) getrennten Gegenstelle (10) über zumindest eine Schnittstelle des Beatmungsgerätes (1) auf das Beatmungsgerät (1) übertragen werden und die Modulaktualisierungen auf dem Beatmungsgerät (1) durchgeführt werden.

## Beschreibung

Die Erfindung betrifft ein Verfahren zur Aktualisierung eines Beatmungsgerätes.

Moderne Beatmungsgeräte weisen eine Vielzahl an verbauter Elektronikbauteile auf, welche zu großen Teilen programmierbar sind und über Firmware und Software betrieben werden. Mitunter werden neue Funktionen für diese Bauteile entwickelt oder Fehler in der ursprünglichen Programmierung behoben. Um diese Änderungen auf bereits vorhandene Beatmungsgeräte übertragen zu können, muss die Firmware und Software der Beatmungsgeräte aktualisiert werden.

Häufig wird die Firmware und Software der Beatmungsgeräte des Stands der Technik mit einem großen Update als Ganzes aktualisiert oder das Beatmungsgerät wird komplett mit einer neuen Firmware- und Software-Version überspielt. Die Übertragung und Installation solcher Aktualisierungen nimmt dabei viele Ressourcen, unter anderem auch viel Zeit, in Anspruch. Wird die Übertragung unbeabsichtigt unterbrochen, kann es dazu führen, dass bereits übertragene Teile der Aktualisierung vollständig verloren gehen und neu übertragen werden müssen, was die Aktualisierung weiter hinauszögert.

Aufgabe der vorliegenden Erfindung ist daher die Bereitstellung eines Verfahrens zum sicheren Betreiben eines Beatmungsgeräts. Die Aufgabe wird durch ein Verfahren nach Anspruch 1 sowie durch das System nach Anspruch 16 gelöst.

Verfahren zur Aktualisierung der Firmware und Software eines Beatmungsgerätes, wobei die Firmware und die Software des Beatmungsgerätes in einzelne Module unterteilt ist und zu jedem Modul separate Aktualisierungen in Form von Modulaktualisierungen bereitgestellt werden können und diese Modulaktualisierungen von zumindest einer räumlich von dem Beatmungsgerät getrennten Gegenstelle über zumindest eine Schnittstelle des Beatmungsgerätes auf das Beatmungsgerät übertragen werden können und die Modulaktualisierungen auf dem Beatmungsgerät durchgeführt werden können.

In manchen Ausführungsformen des Verfahrens sind die Modulaktualisierungen jeweils in Datenpakete unterteilt, welche über eine Schnittstelle des Beatmungsgerätes auf das Beatmungsgerät übertragen und gespeichert werden, wobei die Datenpakete nach Übertragung aller Datenpakete einer Modulaktualisierung zusammengesetzt werden und die jeweilige Modulaktualisierung durchgeführt wird.

In manchen Ausführungsformen des Verfahrens werden die Datenpakete einer Modulaktualisierung auch nach Unterbrechung der Datenübertragung auf dem Beatmungsgerät gespeichert.

In manchen Ausführungsformen des Verfahrens wird nach einer Unterbrechung der Datenübertragung die Übertragung der Datenpakete fortgesetzt.

In manchen Ausführungsformen des Verfahrens wird nach einer Unterbrechung der Datenübertragung die Übertragung der Datenpakete mit fortgesetzt, wobei mit dem zuletzt begonnenen, nicht vollständig übertragenen Datenpaket wieder begonnen wird.

In manchen Ausführungsformen des Verfahrens werden die Datenpakete einer Modulaktualisierung solange auf dem Beatmungsgerät gespeichert bis die Modulaktualisierung durchgeführt wurde oder ein Befehl zum Löschen der Datenpakete erfolgt.

In manchen Ausführungsformen des Verfahrens werden die übertragenen Datenpakete auf dem Beatmungsgerät einer nicht vollständig übertragenen Modulaktualisierung verworfen/gelöscht und die fehlenden Datenpakete nicht übertragen werden, wenn eine neuere Version der Modulaktualisierung zur Verfügung steht, wobei dann die Datenpakete der neuen Version der Modulaktualisierung übertragen werden.

In manchen Ausführungsformen des Verfahrens wird eine Meldung über den Status der Modulaktualisierung von dem Beatmungsgerät und/oder der räumlich getrennten Gegenstelle erzeugt und/oder ausgegeben.

In manchen Ausführungsformen des Verfahrens muss die Übertragung und/oder Durchführung der Modulaktualisierung an dem Beatmungsgerät und/oder der räumlich getrennten Gegenstelle angefordert oder bestätigt werden.

In manchen Ausführungsformen des Verfahrens wird die Übertragung der Modulaktualisierung bzw. der Datenpakete unter bestimmten Voraussetzungen ausgesetzt.

In manchen Ausführungsformen des Verfahrens wird eine Übertragung von medizinischen, patientenspezifischen und/oder therapiebezogenen Daten gegenüber der Übertragung von Datenpaketen der Modulaktualisierungen priorisiert.

In manchen Ausführungsformen des Verfahrens ist die Priorität der Übertragung am Beatmungsgerät und/oder an der räumlich getrennten Gegenstelle für einzelne Datenarten (z.B. medizinische, patientenspezifische und/oder therapiebezogene Daten; Modulaktualisierungen) individuell einstellbar.

In manchen Ausführungsformen des Verfahrens wird die Übertragung und Durchführung einer Modulaktualisierung unter bestimmten Umständen allen anderen Datenübertragungen vorgezogen.

In manchen Ausführungsformen des Verfahrens sind die Modulaktualisierungen in Datenpakete unterteilt und die maximale Größe der Datenpakete beträgt von 100 Kilobyte bis 100000 Kilobyte. In manchen Ausführungsformen beträgt die maximale Größe der Datenpakete zwischen 100 Kilobyte und 1000 Kilobyte.

In manchen Ausführungsformen des Verfahrens wird bei der Verfügbarkeit einer neueren Version der Modulaktualisierung zunächst geprüft, ob eine der vorhergehenden Modulaktualisierungen notwendig ist, damit die neue Version der Modulaktualisierung durchgeführt werden kann, wobei dann erst die ältere Version der Modulaktualisierung übertragen und durchgeführt wird, bevor die neuere Version der Modulaktualisierung übertragen und durchgeführt wird.

In manchen Ausführungsformen des Verfahrens werden bei einer Vielzahl von verfügbaren Modulaktualisierungen für verschiedene Module die Modulaktualisierungen nach einander übertragen und durchgeführt, wobei die nächste Modulaktualisierung erst dann übertragen und durchgeführt wird, wenn die vorherige Modulaktualisierung vollständig abgeschlossen ist.

In manchen Ausführungsformen des Verfahrens werden bei einer Vielzahl von verfügbaren Modulaktualisierungen für verschiedene Module die Modulaktualisierungen gleichzeitig übertragen und installiert.

In manchen Ausführungsformen des Verfahrens werden bei einer Vielzahl von verfügbaren Modulaktualisierungen für verschiedene Module die Modulaktualisierungen nacheinander übertragen und durchgeführt, wobei die mit der Übertragung der nächsten Modulaktualisierung begonnen wird, nachdem die Übertragung der vorhergehenden Modulaktualisierung abgeschlossen ist und wobei nicht die erfolgreiche Durchführung dieser vorhergehenden Modulaktualisierung abgewartet wird.

In manchen Ausführungsformen des Verfahrens die Übertragung der Modulaktualisierung basierend auf der Verbindung zwischen dem Beatmungsgerät und der räumlich getrennten Gegenstelle automatisch durch das Beatmungsgerät unterbrochen wird.

In manchen Ausführungsformen des Verfahrens findet eine Übertragung der Modulaktualisierung bei einer Verbindung zwischen dem Beatmungsgerät und der räumlich getrennten Gegenstelle über eine Mobilfunkverbindung (LTE, 3G, etc.) nicht statt und wird unterbrochen, falls die Verbindung zwischen dem Beatmungsgerät und der räumlich getrennten Gegenstelle auf eine solche Mobilfunkverbindung wechselt.

Das System zur Beatmung umfasst zumindest ein Beatmungsgerät (1) und eine räumlich getrennte Gegenstelle (10), wobei die Firmware und die Software des Beatmungsgerätes (1) in einzelne Module (20, 30, 40, 50, 60, 70, 80, 90) unterteilt ist und zu jedem Modul (20, 30, 40, 50, 60, 70, 80, 90) separate Aktualisierungen in Form von Modulaktualisierungen bereitgestellt werden, diese Modulaktualisierungen von der Gegenstelle (10) über zumindest eine Schnittstelle des Beatmungsgerätes (1) auf das Beatmungsgerät (1) übertragen werden und die Modulaktualisierungen auf dem Beatmungsgerät (1) durchgeführt werden.

Es ist darauf hinzuweisen, dass die in den Ansprüchen einzeln aufgeführten Merkmale in beliebiger, technisch sinnvoller Weise miteinander kombiniert werden können und weitere Ausgestaltungen der Erfindung aufzeigen. Die Beschreibung charakterisiert und spezifiziert die Erfindung insbesondere im Zusammenhang mit den Figuren zusätzlich.

Es sei ferner darauf hingewiesen, dass eine hierin verwendete, zwischen zwei Merkmalen stehende und diese miteinander verknüpfende Konjunktion "und/oder" stets so auszulegen ist, dass in einer ersten Ausgestaltung des erfindungsgemäßen Gegenstands lediglich das erste Merkmal vorhanden sein kann, in einer zweiten Ausgestaltung lediglich das zweite Merkmal vorhanden sein kann und in einer dritten Ausgestaltung sowohl das erste als auch das zweite Merkmal vorhanden sein können.

Unter einem Beatmungsgerät ist im Sinne der Erfindung ist jedwedes Gerät zu verstehen, welches zu medizinischen Zwecken eigesetzt werden kann. Diese medizinischen Zwecke können zum Beispiel die Therapie eines Patienten, die Diagnose von Krankheiten und Leiden eines Patienten oder auch die Unterstützung der Lebensfunktionen sein. Insbesondere können Beatmungsgeräte in Form von Beatmungsgeräten und Diagnosegeräten verstanden werden. Diagnosegeräte können dabei allgemein zur Erfassung von medizinischen Parametern eines Patienten dienen. Darunter fallen auch Geräte, welche medizinische Parameter von Patienten in Kombination mit oder ausschließlich die Atmung betreffend, erfassen und optional verarbeiten können. Unter einem Beatmungsgerät ist jedwedes Gerät zu verstehen, welches einen Anwender oder Patienten bei der natürlichen Atmung unterstützt, die Beatmung des Anwenders bzw. Patienten übernimmt und/oder zur Atemtherapie dient und/oder anderweitig die Atmung des Anwenders bzw. Patienten beeinflusst. Darunter fallen zum Beispiel, aber nicht ausschließend, CPAP- sowie BiPAP-Geräte, Narkose- bzw. Anästhesiegeräte, Atemtherapiegeräte, (klinische, außerklinische oder Notfall-) Beatmungsgeräte, Highflow-Therapiegeräte und Hustenmaschinen.

Eine Schnittstelle ist im weitesten Sinne als jede Art von Einrichtung zur Verbindung - unabhängig der Verbindungsart - zwischen dem Beatmungsgerät und einer Gegenstelle oder einer Person zu verstehen. Eine Schnittstelle für eine Verbindung zwischen dem Beatmungsgerät und einer Person kann dabei zum Beispiel eine Benutzerschnittstelle sein, welche die Interaktion zwischen Person und Beatmungsgerät direkt am Beatmungsgerät ermöglicht.

Die einzelnen Bestandteile des Beatmungsgeräts sind in Module, wobei auch das gesamte Beatmungsgerät als ein einziges Modul verstanden werden kann. Die Definition eines Moduls kann unter verschiedenen Gesichtspunkten erfolgen, welche an dieser Stelle nicht näher erläutert werden und keinen Einfluss auf die Erfindung als solche haben. Auch ein Überschneiden von verschiedenen Modulen durch die Bestandteile des Beatmungsgeräts ist dabei möglich. So kann ein Bestandteil beispielsweise zu zwei Modulen zugeordnet sein. Weiter können die einzelnen Module auch zu Modulgruppen zusammengefasst werde, wobei auch diese Modulgruppen unter Umständen Überschneidungen aufweisen können.

Wird in manchen Ausführungsformen eine Modulaktualisierung nicht vollständig übertragen muss zunächst geprüft werden, ob eine neuere Modulaktualisierung verfügbar ist. Ist eine neuere Modulaktualisierung verfügbar, muss festgestellt werden, ob die neue Modulaktualisierung streng auf der vorherigen Aktualisierung aufbaut oder eigenständig (versionsübergreifend) installiert werden kann. Baut die Modulaktualisierung auf der vorherigen Version auf, muss zunächst die vorherige Version übertragen und installiert werden. Ist dies nicht der Fall, so können die Daten der unvollständigen Modulaktualisierung gelöscht werden und die Übertragung der neuen Modulaktualisierung begonnen werden.

In manchen Ausführungsformen des Verfahrens wird die Übertragung der Modulaktualisierung ausgesetzt oder verschoben, wenn derzeit eine andere Datenübertragung vorgesehen ist. Dies ist beispielsweise bei Verbindungen über Mobilfunk wichtig, da diese häufig teuer, schwach und/oder Volumen-beschränkt sind. Die Wichtigkeit der Modulaktualisierung kann also als untergeordnet wichtig angesehen werden. In manchen Fällen kann die Übertragung von Modulaktualisierungen über Mobilfunkverbindung auch völlig ausgesetzt werden.

Gelegentlich kann es zu wichtigen Aktualisierungen kommen, z.B. um Sicherheitslücken zu schließen. Diese können beispielsweise vom Server aus als wichtig angegeben werden, sodass das Beatmungsgerät beispielsweise einen Befehl bekomm, welcher beinhaltet, dass die Modulaktualisierung sofort übertragen und installiert werden soll. Dies kann in manchen Ausführungsformen auch als so wichtig eingestuft werden, dass ohne die entsprechende Modulaktualisierung der Betrieb des Beatmungsgerätes nicht mehr möglich ist.

Die Firmware des Beatmungsgeräts ist dabei beispielsweise in einzelne Module unterteilt, welche jeweils einzeln durch neuere Versionen der Firmware aktualisiert werden können. Die Modularität der Firmware ermöglicht dabei, dass nur die jeweils neue Firmware eines einzelnen Moduls übertragen werden. Dadurch werden die Aktualisierungen in ihrem Umfang kleiner und ergeben auch potentiell die Möglichkeit, schneller Aktualisierungen für einzelne Module bereitzustellen anstatt jeweils ein größeres Update für die ganze Firmware bereitzustellen.

Die neue Version der jeweiligen Modul-Firmware wird beispielsweise wiederum in mehrere kleinere Datenpakete aufgeteilt, welche auf das Beatmungsgerät übertragen werden und dort (zwischen)gespeichert werden. Wird die Übertragung unterbrochen, so kann zu einem späteren Zeitpunkt wieder mit der Übertragung der noch fehlenden Pakete fortgesetzt werden. Sobald alle Pakete eines Updates übertragen sind, werden diese zur vollständigen Modulaktualisierung zusammengesetzt und das Gerät kann die Aktualisierung durchführen.

Sollte beispielsweise eine längere Zeit keine Verbindung des Beatmungsgeräts zustande kommen, kann es vorkommen, dass zwischenzeitlich eine neuere als die noch nicht fertig übertragene Version der Modulaktualisierung bereitgestellt wurde. Ist dies der Falls können die bisher übertragenen Pakete der noch nicht fertig übertragenen Version verworfen werden und mit der Übertragung der Pakete der neuen Version begonnen werden. Sollte die neuere Version der Modulaktualisierung auf der älteren Version aufbauen, also davon abhängig sein, so kann auch zunächst die ältere Version fertig übertragen und durchgeführt werden und im Anschluss mit der neuen Version fortgefahren werden.

In manchen Ausführungsformen des Verfahrens wird vor der Modulaktualisierung die aktuelle Bandbreite und/oder Stabilität der Schnittstelle überprüft. Die trifft insbesondere bei Mobilfunkverbindungen zu. Beispielsweise wird die Aktualisierung wird nur durchgeführt bzw. fortgesetzt, wenn die Bandbreite und/oder Stabilität der Verbindung zwischen Beatmungsgerät und Gegenstelle einen Mindestwert überschreitet.

Für die einzelnen Module wird in manchen Ausführungsformen eine Priorität für die Aktualisierung der Firmware und/oder Software vergeben. Diese kann sich z. B. nach der Größe der Aktualisierungsdatei richten oder nach der Relevanz von durch die Aktualisierung zu behebenden Fehlern oder neuen Funktionen. Die Priorität kann beispielsweise auch anhand logischer Abhängigkeiten der einzelnen Module erfolgen, z.B. wenn die aktualisierte Version eines Moduls nur unter der Voraussetzung ausführbar ist, dass zuvor ein anderes Modul aktualisiert wurde. Die Aktualisierung aller Module erfolgt dann in der Reihenfolge der Priorität. Bei einer geringen Bandbreite und/oder Stabilität der Verbindung werden zunächst oder ausschließlich die Module mit einer hohen Priorität übertragen.

In manchen Ausführungsformen des Verfahrens kann sich die Unterteilung der gesamten Firmware und/oder Software in Module an der Modularisierung der Hardware orientieren. In diesem Fall wird ein Firmware-/Softwaremodul pro programmierbarem Elektronikbauteil, z.B. Mikroprozessor, Funkmodul, Speichermodul, Schnittstellenmodul, Sensormodul etc. erstellt und zumindest optional einzeln aktualisiert. Die Unterteilung kann beispielsweise auch innerhalb eines Elektronikbauteils erfolgen, z.B. in mindestens ein Betriebssystem-Modul und mindestens ein Applikationsmodul, welches das Betriebssystem-Modul benötigt, um ausgeführt werden zu können. Zusätzlich zu mindestens einem Applikationsmodul kann auch mindestens ein separates Sprachmodul existieren, in dem Schriftzeichen und/oder AnzeigeTexte abgelegt sind.

In manchen Ausführungsformen erfolgt die Aktualisierung nur während dafür geeigneten Betriebszuständen des Beatmungsgerätes, z.B. nicht während aktiver Therapie- oder Diagnosefunktionen.

In manchen Ausführungsformen kann über die Schnittstelle auch ein Kommando/Befehl an das Beatmungsgerät gesendet werden, welches eine weitere Verwendung erst nach erfolgter Aktualisierung erlaubt, insbesondere falls die Ausgang-Version des Beatmungsgerätes bzw. der Modulfirmware/Modulsoftware kritische Fehler enthält.

Die Auswahl der Version, auf die die einzelnen Module aktualisiert werden, kann beispielsweise anhand des Standortes des Beatmungsgerätes erfolgen, insbesondere des Landes. Die Auswahl kann insbesondere auf Basis der Zulassungssituation im betreffenden Land erfolgen. Insbesondere wird die neueste im betreffenden Land zugelassene Version für die Aktualisierung angeboten oder empfohlen. Der Standort kann z. B. mittels GPS, IP-Adresse, Betreiber eines Mobilfunknetzes oder Einwahl-Zelle in einem Mobilfunknetz, Sprachauswahl oder durch Benutzereingabe erfolgen.

In manchen Ausführungsformen übermittelt das Beatmungsgerät vor der Aktualisierung seine Elektronik- und/oder Mechanik-Konfiguration, z.B. Vorhandensein oder Version von Schnittstellen- oder Sensormodulen oder sonstiger wesentlicher Komponenten. Anhand der Konfiguration werden die benötigten Module sowie deren Version für die Aktualisierung ausgewählt.

In manchen Ausführungsformen muss die Aktualisierung der Firmware und/oder Software am Beatmungsgerät extra bestätigt werden. So kann beispielsweise verhindert werden, dass eine speziell angepasste Firmware oder Software durch eine allgemeine Aktualisierung überspielt und unbrauchbar wird.

In manchen Ausführungsformen werden Modulaktualisierungen und/oder einzelne Datenpakete hinsichtlich ihrer Gültigkeit geprüft. Eine ungültige Modulaktualisierung und/oder ein ungültiges Datenpaket kann beispielsweise sein, wenn keine oder eine andere Aktualisierung angefordert wird und/oder eine Checksumme/Prüfsumme nicht stimmt und/oder eine falsche bzw. fehlende Verschlüsselung der Daten und/oder eine fehlende bzw. falsche Signatur der Daten.

In manchen Ausführungsformen kann vorgesehen sein, dass die einzelnen Datenpakete nach der Übertragung auf das Beatmungsgerät zunächst auf Gültigkeit geprüft werden. Dabei wird beispielsweise geprüft, ob eine fehlerfreie Übertragung stattgefunden hat. Auch kann dabei überprüft werden, ob das übertragene Datenpaket tatsächlich zu dem Modul und/oder der Modulaktualisierung und/oder der zu installierenden Version gehört. Eine solche Prüfung kann beispielsweise anhand einer Checksumme oder über ähnliche Prüfungen erfolgen.

Es kann auch vorgesehen sein, dass nach dem Empfang aller Datenpakete, die Datenpakete zusammengesetzt werden, beispielsweise im Speicher des Gerätes. Nach dem Zusammensetzen der Datenpakete zur Modulaktualisierung kann eine weitere Prüfung, beispielsweise auf Vollständigkeit, Gültigkeit und/oder ob die Modulaktualisierung zum Modul passt, durchgeführt werden. Erst wenn diese Prüfung erfolgreich ist, wird die Modulaktualisierung installiert. Eine Installation kann beispielsweise das Laden des Programmcodes in den Programmspeicher und anschließende Aktivierung.

In manchen Ausführungsformen werden die Datenpakete bei bzw. vor der Übertragung verschlüsselt und/oder digital signiert. Es kann dazu auch vorgesehen sein, dass die gesamte Modulaktualisierung verschlüsselt und/oder digital signiert wird. Wird die Modulaktualisierung und/oder die einzelnen Datenpakete digital signiert, werden nur solche Datenpakete/Modulaktualisierungen vom Beatmungsgerät akzeptiert und/oder installiert, welche über eine gültige Signatur verfügen. Gleiches gilt für die Verschlüsselung von Modulaktualisierungen, welche nur dann aktiviert bzw. installiert werden, wenn ein gültiger Schlüssel vorhanden ist.

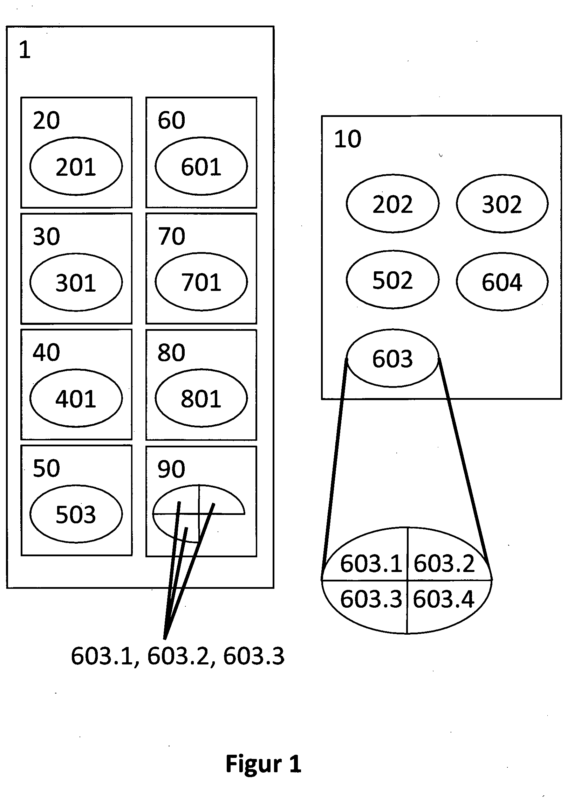

Das erfindungsgemäße Verfahren wird anhand der Figur 1 in einer beispielhaften Ausführungsform näher beschrieben.

Figur 1 zeigt ein Beatmungsgerät 1 dessen Firmware und Software in verschiedene Module unterteilt ist, sowie die räumlich getrennte Gegenstelle 10. Beispielhaft sind dem Beatmungsgerät 1 ein Sensormodul 20, ein Aufbereitungsmodul 30, ein Erkennungsmodul 40, ein Steuerungsmodul 50, ein Kommunikationsmodul 60, ein Überwachungsmodul 70, ein Softwaremodul 80 sowie ein Speichermodul 90 zugeordnet. Die Modularisierung der Firmware und Software kann sich beispielsweise an der Modularisierung der Hardware orientieren. Zum Beispiel wird jedem Hardwaremodul ein entsprechendes Firmware-und/oder Softwaremodul zugeordnet. Weiter ist es auch denkbar, dass eine Unterteilung je nach Elektronikbauteil erfolgt, jedes Elektronikbauteil also beispielsweise in ein Betriebssystemmodul und ein Applikationsmodul unterteilt wird. Dabei kann es beispielsweise so eingerichtet sein, dass das Applikationsmodul das Betriebssystemmodul benötigt um ausgeführt zu werden.

Das Sensormodul 20 ist eingerichtet, Messwerte, insbesondere Parameter, die mit einem Atemfluss, einem Atemvolumen, einer Atemfrequenz, einer Einatmungs- und Ausatmungsdauer, einer Atemkontur, einer Leckage oder einem Therapiedruck in Zusammenhang stehen, zu erfassen. Optional kann das Sensormodul 20 zusätzliche Messungen von Bestandteilen oder Temperatur des Atemgases oder des Blutes vornehmen. Das Sensormodul 20 übermittelt die erfassten Messwerte an das Aufbereitungsmodul 30.

Das Aufbereitungsmodul 30 kann die erfassten Messwerte aufbereiten. Beispielsweise kann das Aufbereitungsmodul 30 eine Glättung, eine Artefaktbereinigung oder ein Downsampling der Messwerte durchführen. In manchen Ausführungsformen ist das Aufbereitungsmodul 30 auch als kombiniertes Aufbereitungs- und Berechnungsmodul ausgebildet, alternativ oder ergänzend können diese Modulen auch als separate Modulen ausgebildet sein. Das Berechnungsmodul berechnet aus den durch das Sensormodul erfassten und durch das Aufbereitungsmodul aufbereiteten Messwerten Signale und/oder Kenngrößen, wie beispielsweise einen Mittelwert, einen Median, ein Perzentil, eine Ableitung, eine Häufigkeitsverteilung, eine Dauer oder einen Anteil eines Über- oder Unterschreitens von Schwellenwerten.

Das Erkennungsmodul 40 ist eingerichtet, Ereignisse/Zustände wie beispielsweise Alarme, Atemaussetzer, Artefakte, Hustenstöße, Sauerstoff(ent)sättigungen, asynchrone zwischen Gerät und Anwender, Einatmen, Ausatmen oder mandatorische Atemzüge zu erkennen.

Das Steuermodul 50 dient beispielsweise zur Steuerung des Beatmungsgerätes 1, insbesondere eines hier nicht abgebildeten Gebläse- und/oder Ventilmodul zur Erzeugung des Atemgasstroms bzw. Beatmungsdruckes. Auch kann das Steuermodul 50 dazu ausgebildet sein, andere Bestandteile und/oder Module des Beatmungsgeräte 1 zu steuern. In manchen Ausführungsformen kann das Steuermodul 50 auch weiter unterteilt sein und aus mehreren Steuermodulen bestehen, welche jeweils ein individuelles Modul und/oder Bestandteil des Beatmungsgerätes 1 steuern.

Das Kommunikationsmodul 60 ist so eingerichtet, dass sich das Beatmungsgerät 1 darüber zu räumlich getrennten Gegenstellen, wie zum Beispiel einem Server, verbinden und mit dieser Gegenstelle Daten austauschen, also von der Gegenstelle Daten empfangen und an die Gegenstelle Daten senden, kann. Das Kommunikationsmodul 60 dient also unter anderem als Schnittstelle zur Gegenstelle 10. Beispielsweise ist das Kommunikationsmodul 60 als Schnittstelle für folgende Kommunikationssysteme ausgebildet und eingerichtet: GSM, Lora, LPWAN, NB-IOT, LTE-M, LTE, UMTS, LAMN WIFI, WLAN, Bluetooth, LAN, WPAN. Darüber hinaus kann das Kommunikationsmodul 60 auch als Schnittstelle für kabelgebundene Datenverbindung oder auch für Datenspeicher wie USB-Sticks und SD-Karten dienen.

Das Überwachungsmodul 70 erfasst zum Beispiel technische Probleme des Beatmungsgerätes 1. Technische Probleme können beispielsweise ein niedriger Akkustand, Fehler in der Elektronik, ein defekter Akku, ein defektes Bauteil, ein Stromausfall, ein nicht korrekt funktionierendes Zubehörteil, ein unplausibler Messwert oder ein Verlassen eines erlaubten Temperaturbereichs sein. Das Überwachungsmodul 70 kann bei einem erkannten technischen Problem einen Alarm auf dem Beatmungsgerät 1 über eine Schnittstelle anzeigen oder übermitteln.

Das Speichermodul 90 speichert unter anderem die durch das Sensormodul 20 erfassten Werte/Parameter und/oder die durch das Aufbereitungsmodul 30 aufbereiteten Werte, Daten und/oder Informationen, beziehungsweise speichert diese zumindest zwischen. Eine Zwischenspeicherung bedeutet zum Beispiel, dass die Werte, Daten und/oder Informationen bis zu einer Übertragung gespeichert und danach zum Beispiel gelöscht oder zum Überschreiben freigegeben werden. Werden Modulaktualisierungen auf das Beatmungsgerät 1 übertragen, so werden diese in dem Speichermodul 90 zumindest zwischengespeichert.

Die Gegenstelle 10 kann jede von dem Beatmungsgerät 1 räumlich getrennte Datenquelle sein, wie zum Beispiel ein Server, zu welchem das Kommunikationsmodul 60 eine Verbindung aufbauen kann. Die Gegenstelle 10 kann aber auch zum Beispiel ein USB-Stick oder eine SD-Karte oder ein anderes Speichermedium sein. Beispielsweise kann auch ein Smartphone und/oder ein anderes Bluetooth-fähiges Medium als Gegenstelle 10 angesehen werden.

Den Modulen ist jeweils eine Firmware und/oder Software zugeordnet. Beispielhaft sind die jeweiligen Firmware- und/oder Softwareversionen durch die dreistelligen Bezeichnungsnummern gekennzeichnet. Die ersten beiden Ziffern stellen dabei das Modul dar, zu welchem die Firmware- und/oder Softwareversion gehört, die dritte Stelle gibt dabei eine beispielhafte Versionsnummer an, wobei eine höhere Nummer eine aktuellere Version angibt. So sind ist die Sensorfirmware 201 und 202 dem Sensormodul zuzuordnen. Die Sensorfirmware 202 ist dazu die neuere Version der Firmware gegenüber der Sensorfirmware 201. Aus Gründen der Übersicht wurde dem Speichermodul 90 keine Firmware zugeordnet, die Datenpakete 603.1, 603.2 und 603.3 sind dem Speichermodul 90 zugeordnet, wodurch beispielhaft eine Speicherung der Datenpakete im Speichermodul 90 dargestellt werden soll.

Aktualisierungen für die Firmware und/oder Software werden beispielsweise über die Gegenstelle 10 bereitgestellt und über die Schnittstelle/Schnittstellen des Kommunikationsmoduls 60 auf das Beatmungsgerät 1, beziehungsweise das Speichermodul 90 des Beatmungsgerätes 1, übertragen.

In der in Figur 1 beispielhaft dargestellten Situation werden über die Gegenstelle 10 Modulaktualisierungen für das Sensormodul 20, das Aufbereitungsmodul 30, das Steuerungsmodul 50 sowie für das Kommunikationsmodul 60 bereitgestellt. Für das Sensormodul 20 des Beatmungsgerätes 1 ist derzeit die Sensorfirmware 201 installiert, die neuere Sensorfirmware 202 wird von der Gegenstelle 10 als Modulaktualisierung bereitgestellt. Das Aufbereitungsmodul 30 läuft mit der Aufbereitungsfirmware 301 und die Gegenstelle stellt auch hierfür eine neuere Aufbereitungsfirmware 302 bereit. Zudem ist für das Steuerungsmodul 50 die Steuerungsfirmware 503 installiert, die Gegenstelle 10 stellt hier die ältere Steuerungsfirmware 502 zur Verfügung. Zudem stellt die Gegenstelle 10 noch die Kommunikationsfirmware 603 und 604 für das Kommunikationsmodul 60 bereit, wobei auf dem Beatmungsgerät 1 die ältere Kommunikationsfirmware 601 installiert ist.

Wird eine Verbindung zwischen der Gegenstelle 10 und dem Beatmungsgerät1 aufgebaut, so wird zunächst unter anderem geprüft, welche Firmware auf dem Beatmungsgerät 1 installiert ist. Die Prüfung kann beispielsweise durch die Gegenstelle 10 stattfinden, etwa dadurch, dass das Beatmungsgerät 1 Informationen zu der installierten Firmware an die Gegenstelle 10 sendet und die Gegenstelle 10 überprüft, ob neuere Versionen zu einer der Firmware in Form einer Modulaktualisierung verfügbar ist. In manchen Ausführungsformen kann auch durch das Beatmungsgerät 1 geprüft werden, ob neuere Versionen der Firmware verfügbar ist. Beispielsweise sendet dazu die Gegenstelle 10 Informationen zur verfügbaren Firmware und das Beatmungsgerät 1 gleicht diese mit der installierten Firmware ab. Ist die Gegenstelle beispielsweise eine SD-Karte oder ein USB-Stick kann das Beatmungsgerät 1 auch den Inhalt der SD-Karte auf Modulaktualisierungen, also neuere Firmware, prüfen.

Zusätzlich wird beispielsweise auch die verfügbare/aktuelle Bandbreite (Übertragungsgeschwindigkeit) und die Stabilität der Verbindung vor Übertragung und Durchführung der Modulaktualisierung geprüft. Dies geschieht insbesondere bei Mobilfunkverbindungen. Dadurch wird zum Beispiel sichergestellt, dass es bei der Übertragung zu keinen Störungen kommt oder aber auch ob die Bandbreite/Übertragungsgeschwindigkeit ausreichend ist um sowohl therapeutische/medizinische Daten und Einstellungen zwischen Gegenstelle 10 und Beatmungsgerät 1 zu übertragen als auch die nötigen Daten zur Modulaktualisierung zu übertragen. Beispielsweise wird auch während der Übertragung beziehungsweise Durchführung der Modulaktualisierung die Verbindung zur Gegenstelle 10 überprüft und nur fortgesetzt, wenn die Stabilität und/oder die Bandbreite einen Mindestwert überschreitet.

Sind Modulaktualisierungen verfügbar beziehungsweise werden von der Gegenstelle 10 bereitgestellt, wird die jeweilige Modulaktualisierung auf das Beatmungsgerät 1 übertragen und in beispielsweise dem Speichermodul 90 zumindest zwischengespeichert. Ist die Modulaktualisierung vollständig auf das Beatmungsgerät 1 übertragen, kann diese installiert, also die Aktualisierung durchgeführt werden.

In dem in Figur 1 beispielhaft dargestellten Fall würde zumindest für das Sensormodul 20 und das Aufbereitungsmodul 30 die jeweiligen Modulaktualisierungen übertragen und durchgeführt, sodass nach Abschluss der Aktualisierung auf dem Beatmungsgerät 1 die Sensorfirmware 202 und die Aufbereitungsfirmware 302 installiert sind. Für das Steuerungsmodul 50 wird keine Modulaktualisierung übertragen und durchgeführt, da bereits eine neuere Steuerungsfirmware 503 installiert ist und die Gegenstelle 10 nur die ältere Steuerungsfirmware 502 bereitstellt. Ein solcher Fall kann beispielsweise auftreten, wenn das Beatmungsgerät 1 zuvor per Internetverbindung eine Modulaktualisierung für das Steuerungsmodul 50 erhalten hat und nun über beispielsweise eine SD-Karte aktualisiert werden soll, welche allerdings nur die ältere Steuerungsfirmware 502 enthält.

Beispielsweise werden die Modulaktualisierungen nacheinander übertragen, das heißt, dass zunächst eine Modulaktualisierung vollständig übertragen wird bevor mit der Übertragung der nächsten Modulaktualisierung begonnen wird. Sind mehrere Modulaktualisierungen verfügbar kann beispielsweise anhand einer Priorität der Modulaktualisierung entschieden werden, welche zuerst übertragen wird. Wird die Sensorfirmware 202 beispielsweise gegenüber der Aufbereitungsfirmware 302 priorisiert, so wird zunächst die Modulaktualisierung zu Sensorfirmware 202 auf das Beatmungsgerät übertragen. In manchen Ausführungsformen wird dabei mit der Übertragung der Aufbereitungsfirmware 302 gewartet bis die Modulaktualisierung zu Sensorfirmware 202 abgeschlossen ist. Es kann aber auch möglich sein, dass bereits mit der Übertragung der nächsten Modulaktualisierung begonnen wird, nachdem die Übertragung der vorherigen abgeschlossen ist und diese zum Beispiel gerade ausgeführt wird. In manchen Ausführungsformen kann es auch möglich sein, dass alle Modulaktualisierungen gleichzeitig übertragen werden. Auch eine gleichzeitige Installation der Modulaktualisierungen ist durchaus denkbar.

Ob eine Modulaktualisierung übertragen und durchgeführt/installiert wird kann in manchen Ausführungsformen beispielsweise von den verfügbaren Ressourcen wie Speicherplatz im Speichermodul 90, verfügbare Prozessorleistung, laufende Therapie/Diagnose, Datenverbindung, Akkustand beziehungsweise Stromversorgung und/oder anderen laufenden Datenübertragungen abhängen. Eine Modulaktualisierung kann so zum Beispiel ausgesetzt oder verschoben werden, sollte das Speichermodul 90 nicht ausreichend Speicherplatz aufweisen, um die Modulaktualisierung speichern zu können. Insbesondere während einer laufenden Therapie mit dem Beatmungsgerät 1 kann es möglich sein, dass grundsätzlich keine Modulaktualisierungen vorgenommen werden. Zum einen wäre denkbar, dass bei der Durchführung der Aktualisierung der Prozessor überlastet wird und somit das Beatmungsgerät 1 ausfällt oder auch, dass ein aktives und/oder für die Therapie notwendiges Modul aktualisiert werden soll, welches während der Aktualisierung eventuell kurzzeitig nicht zur Verfügung steht und somit die Therapie stören würde.

In manchen Ausführungsformen können die Modulaktualisierungen zurückgestellt werden, wenn zum Beispiel gerade eine Datenübertragung therapiebezogener oder patientenspezifischer Daten stattfindet. Auch kann eine Übertragung der Modulaktualisierung ausgesetzt und/oder unterbrochen werden, wenn das Beatmungsgerät 1 mit der Gegenstelle 10 über eine Mobilfunkverbindung (LTE, 3G, EDGE, etc.) verbunden ist und/oder die vorhandene Übertragungsgeschwindigkeit niedrig ist beziehungsweise voll ausgelastet ist.

Beispielsweise kann den Modulaktualisierungen eine Priorität zugeordnet werden. Die Priorität kann sich beispielweise an der Größe der Datei der Modulaktualisierung orientieren oder auch nach einer Relevanz von zu behebenden Fehlern und/oder neuen Funktionen. In manchen Fällen kann die Priorität der Modulaktualisierung beispielsweise auch einer logischen Reihenfolge folgen, zum Beispiel, wenn die (aktualisierte) Firmware eines Moduls nur ausführbar ist, wenn zuvor andere Module bereits aktualisiert wurden. Die Übertragung und Installation/Durchführung der Modulaktualisierung erfolgt dabei in der Reihenfolge der Priorität. Bei einer geringen Stabilität und/oder Übertragungsgeschwindigkeit der Verbindung zwischen Beatmungsgerät 1 und Gegenstelle 10 werden dann beispielsweise auch vorrangig zunächst Modulaktualisierungen hoher Priorität übertragen und durchgeführt.

In manchen Ausführungsformen kann beispielsweise über zum Beispiel eine in Figur 1 nicht dargestellte Benutzerschnittstelle entschieden werden, ob und welche der verfügbaren Modulaktualisierungen übertragen und durchgeführt/installiert werden sollen. Auch können am Beatmungsgerät 1 oder über die Gegenstelle 10 manuell entschieden werden, ob und welche Modulaktualisierungen bevorzugt werden sollen. In manchen Ausführungsformen können die Modulaktualisierungen dadurch auch gegenüber therapie- oder patientenbezogenen und medizinischen Daten bei der Datenübertragung priorisiert werden. In manchen Ausführungsformen ist auch eine automatische Modulaktualisierung denkbar, welche am Beatmungsgerät 1 aktiviert und deaktiviert werden kann. Bei aktiver automatischer Modulaktualisierung werden die Modulaktualisierungen, falls verfügbar, automatisch auf das Beatmungsgerät 1 übertragen und ausgeführt/installiert.

Weiter ist es für manche Ausführungsformen des Verfahrens denkbar, dass bestimmte Modulaktualisierungen auch noch vor der Therapie selbst Vorrang haben. Das heißt, dass in diesen Fällen eine Therapie nicht gestartet werden kann, bevor die Modulaktualisierung übertragen und installiert ist. Diese Modulaktualisierungen können beispielsweise Fehlerbehebungen enthalten, welche den sicheren und einwandfreien Betrieb des Beatmungsgerätes betreffen. Werden beispielsweise nachträglich Fehler und/oder Störungen der Firmware und/oder Software festgestellt, welche zu einer Fehlfunktion des Beatmungsgerätes 1 oder zumindest eines der Module führen kann, kann entschieden werden, dass diese Fehlerbehebung vorrangig, also mit hoher oder höchster Priorität, als Modulaktualisierung zu installieren ist. Auch können Modulaktualisierungen zur Erhöhung der Datensicherheit, zum Beispiel zum Schließen einer Sicherheitslücke, eine besonders hohe, beispielsweise die höchste, Priorität zugeordnet bekommen.

In manchen beispielhaften Ausführungsformen sind die Modulaktualisierungen in kleinere Datenpakete unterteil, wie in Figur 1 schematisch für die Kommunikationsfirmware 601, 603, 604 des Kommunikationsmoduls 60 dargestellt. Beispielhaft ist dargestellt, dass die Modulaktualisierung zur Kommunikationsfirmware 603 in vier einzelne Datenpakete 603.1, 603.2, 603.3, 603.4 unterteilt ist. Diese Datenpakete 603.1, 603.2, 603.3, 603.4 werden einzeln übertragen und nach erfolgreicher Übertragung aller Datenpakete im Beatmungsgerät 1 zur Modulaktualisierung zusammengesetzt. Die Größe der Datenpakete, in welche eine Modulaktualisierung unterteilt werden kann, kann variabel gesetzt werden. Beispielsweise kann die maximale Größe eines Datenpaketes zwischen 100 Kilobyte und 100000 Kilobyte, bevorzugt zwischen 100 Kilobyte und 10000 Kilobyte/oder zwischen 100 Kilobyte und 1000 Kilobyte betragen. Ist von wenigen oder schlechten Datenverbindungen auszugehen, kann beispielsweise eine kleinere maximale Größe gewählt werden, sodass eine Modulaktualisierung in viele kleine Datenpakete unterteilt wird. Um die Prozessorlast bei Zusammensetzten der Datenpakete zu der Modulaktualisierung niedrig zu halten kann die Größe der Datenpakete beispielsweise auch größer, also bis zu 100000 Kilobyte, gewählt werden um weniger Datenpakete zu erhalten.

Für das Kommunikationsmodul 60 ist in Figur 1 beispielhaft die Kommunikationsfirmware 601 installiert. Beispielsweise wurde bereits mit der Übertragung der Modulaktualisierung zu Kommunikationsfirmware 603 begonnen, dabei aber bisher nur die Datenpakete 603.1, 603.2 und 603.3 übertragen und im Speichermodul 90 gespeichert. Das letzte Datenpaket 603.4 ist also noch nicht oder nicht vollständig übertragen, die Modulaktualisierung ist somit noch nicht vollständig übertragen und kann nicht durchgeführt/installiert werden. Wird bei der nächsten Verbindung des Beatmungsgerätes 1 mit einer Gegenstelle 10 zumindest das fehlende Datenpaket 603.4 zur Verfügung gestellt, kann dieses übertragen werden und die Datenpakete zur Modulaktualisierung auf Kommunikationsfirmware 603 zusammengesetzt werden. Die einzelnen Datenpakete bleiben solange im Speichermodul 90 gespeichert, bis zum Beispiel der Aktualisierungsvorgang vollständig abgeschlossen ist und/oder ein expliziter Befehl gegeben wird, die Datenpakete zu löschen. So werden die Datenpakete 603.1, 603.2 und 603.3 nach einem Beenden oder Unterbrechen der Verbindung oder der Datenübertragung zwischen Beatmungsgerät 1 und Gegenstelle 10 nicht gelöscht, sondern bleiben gespeichert.

Beispielhaft stellt die Gegenstelle 10 in Figur 1 auch die aktuellere Version der Kommunikationsfirmware 604 bereit. Ist die Installation der vorhergehenden Version 603 nicht notwendig, sodass die neuere Kommunikationsfirmware 604 funktionieren kann, so wird bei Verbindung zwischen Gegenstelle 10 und Beatmungsgerät 1 das fehlende Datenpaket 603.4 der Modulaktualisierung zu Kommunikationsfirmware 603 nicht übertragen und die bereits übertragenen Datenpakete 603.1, 603.2 und 603.3 werden aus dem Speichermodul 90 des Beatmungsgerätes 1 gelöscht. Stattdessen wird die Modulaktualisierung auf Kommunikationsfirmware 604 beziehungsweise die entsprechenden Datenpakete auf das Beatmungsgerät übertragen und die Modulaktualisierung durchgeführt/installiert.

Ist beispielsweise die Modulaktualisierung auf Kommunikationsfirmware 603 notwendig damit auch die Kommunikationsfirmware 604 für das Kommunikationsmodul 60 funktionsfähig installiert werden kann, so wird hingegen zunächst die Kommunikationsfirmware 603 vollständig übertragen und installiert. Erst nach erfolgreicher Installation der Modulaktualisierung auf Kommunikationsfirmware 603 wird mit der Übertragung der Modulaktualisierung auf Kommunikationsfirmware 604 begonnen. Sollte diese Übertragung unterbrochen werden und die Modulaktualisierung nicht vollständig übertragen und installiert werden und bei der nächsten Verbindung eine gegenüber der Kommunikationsfirmware 604 neuere Version bereitgestellt werden, so wird erneut überprüft, ob die neuere Kommunikationsfirmware auf einer der vorherigen Versionen beziehungsweise der vorherigen Version aufbauend ist, also eine der vorherigen Versionen der Kommunikationsfirmware notwendig ist. Gegebenenfalls wird dann entsprechend zunächst die notwendige, ältere Version übertragen und installiert, bevor die neuere Version übertragen und installiert wird.

Welche Modulaktualisierungen vorgenommen werden und welche Version dabei auf das Beatmungsgerät 1 aufgespielt wird kann beispielsweise auch vom Standort des Gerätes abhängig gemacht werden. Der Standort kann zum Beispiel mittels GPS, IP-Adresse, Betreiber eines Mobilfunknetzes oder Einwahl-Zelle in einem Mobilfunknetz, Sprachauswahl und/oder durch eine Benutzereingabe erfolgen.

In manchen Ausführungsformen des Verfahrens lässt sich der gesamte Aktualisierungsvorgang in 2 Phasen unterteilen: eine Phase, in welcher die Modulaktualisierungen auf das Beatmungsgerät übertragen werden und eine Phase, in welcher die Modulaktualisierung vollständig übertragen ist und die durchgeführt/installiert wird. Während die erste Phase der Übertragung der Modulaktualisierungen generell unterbrochen werden kann, ist eine Unterbrechung in der zweiten Phase, die Installationsphase, nicht immer möglich. Beispielsweise kann es so eingerichtet sein, dass Modulaktualisierungen hoher oder höchster Priorität nicht unterbrochen werden können. In manchen Ausführungsformen kann es beispielsweise auch eingerichtet sein, dass eine Kopie der aktuellen Modulfirmware in dem Speichermodul 90 gespeichert wird, bis die neue Version der Firmware erfolgreich installiert ist. Sollte die Installation der neuen Firmware unterbrochen werden und daher die Modulfirmware nicht weiter funktionsfähig sein, so könnte automatisch oder auch manuell die alte Version wieder reaktiviert werden. In manchen Fällen startet die Modulaktualisierung nach einer Unterbrechung wieder automatisch, beispielsweise beim nächsten Einschalten des Beatmungsgerätes. Alternativ oder ergänzend kann eine Modulaktualisierung bzw. die Installation beispielsweise auch verhindert werden, wenn ein Risiko besteht, dass die Modulaktualisierung nicht erfolgreich beendet werden kann. Dies kann beispielsweise der Fall sein, wenn der Akkustand zu niedrig ist, sodass das Beatmungsgerät vor Beendigung der Installation nicht mehr mit Energie versorgt werden könnte.

Beispielsweise ist das Beatmungsgerät 1 und/oder die Gegenstelle 10 auch dazu eingerichtet Informationen zum Status der Modulaktualisierung, wie zum Beispiel Anzahl der bereits übertragenen Datenpakete, Datenmenge, Modulversion, Fortschritt der Installation, zu generieren und/oder anzuzeigen. Die Anzeigen können dabei beispielsweise in graphischer Form (z.B. als Balkendiagramm) oder auch als alphanumerischer Art sein.

### Bezugszeichenliste

- 1: Beatmungsgerät
- 10: Gegenstelle
- 20: Sensormodul
- 30: Aufbereitungsmodul
- 40: Erkennungsmodul
- 50: Steuerungsmodul
- 60: Kommunikationsmodul
- 70: Überwachungsmodul
- 80: Softwaremodul
- 90: Speichermodul
- 201: Sensorfirmware
- 202: Sensorfirmware
- 301: Aufbereitungsfirmware
- 302: Aufbereitungsfirmware
- 401: Erkennungsfirmware
- 502: Steuerungsfirmware
- 503: Steuerungsfirmware
- 601: Kommunikationsfirmware
- 603: Kommunikationsfirmware
- 603.1: Datenpaket
- 603.2: Datenpaket
- 603.3: Datenpaket
- 603.4: Datenpaket
- 604: Kommunikationsfirmware
- 701: Überwachungsfirmware
- 801: Software

## Patentansprüche

1. Verfahren zur Aktualisierung der Firmware und Software eines Beatmungsgerätes (1) **dadurch gekennzeichnet, dass** die Firmware und die Software des Beatmungsgerätes (1) in einzelne Module (20, 30, 40, 50, 60, 70, 80, 90) unterteilt ist und zu jedem Modul (20, 30, 40, 50, 60, 70, 80, 90) separate Aktualisierungen in Form von Modulaktualisierungen bereitgestellt werden und diese Modulaktualisierungen von zumindest einer räumlich von dem Beatmungsgerät (1) getrennten Gegenstelle (10) über zumindest eine Schnittstelle des Beatmungsgerätes (1) auf das Beatmungsgerät (1) übertragen werden und die Modulaktualisierungen auf dem Beatmungsgerät (1) durchgeführt werden.

2. Verfahren nach zumindest einem der vorhergehenden Ansprüche **dadurch gekennzeichnet, dass** die Modulaktualisierungen jeweils in Datenpakete unterteilt sind, welche über eine Schnittstelle des Beatmungsgerätes auf das Beatmungsgerät übertragen und gespeichert werden, wobei die Datenpakete nach Übertragung aller Datenpakete einer Modulaktualisierung zusammengesetzt werden und die jeweilige Modulaktualisierung durchgeführt wird.

3. Verfahren nach zumindest einem der vorhergehenden Ansprüche **dadurch gekennzeichnet, dass** die Datenpakete einer Modulaktualisierung auch nach Unterbrechung der Datenübertragung auf dem Beatmungsgerät gespeichert werden.

4. Verfahren nach zumindest einem der vorhergehenden Ansprüche **dadurch gekennzeichnet, dass** nach einer Unterbrechung der Datenübertragung die Übertragung der Datenpakete mit fortgesetzt wird, wobei mit dem zuletzt begonnenen, nicht vollständig übertragenen Datenpaket wieder begonnen wird.

5. Verfahren nach zumindest einem der vorhergehenden Ansprüche **dadurch gekennzeichnet, dass** die Datenpakete einer Modulaktualisierung solange auf dem Beatmungsgerät gespeichert werden bis die Modulaktualisierung durchgeführt wurde oder ein Befehl zum Löschen der Datenpakete erfolgt.

6. Verfahren nach zumindest einem der vorhergehenden Ansprüche **dadurch gekennzeichnet, dass** die übertragenen Datenpakete auf dem Beatmungsgerät einer nicht vollständig übertragenen Modulaktualisierung verworfen/gelöscht werden und die fehlenden Datenpakete nicht übertragen werden, wenn eine neuere Version der Modulaktualisierung zur Verfügung steht, wobei dann die Datenpakete der neuen Version der Modulaktualisierung übertragen werden.

7. Verfahren nach zumindest einem der vorhergehenden Ansprüche **dadurch gekennzeichnet, dass** die Übertragung und/oder Durchführung der Modulaktualisierung an dem Beatmungsgerät und/oder der räumlich getrennten Gegenstelle angefordert oder bestätigt werden muss.

8. Verfahren nach zumindest einem der vorhergehenden Ansprüche **dadurch gekennzeichnet, dass** die Übertragung der Modulaktualisierung bzw. der Datenpakete unter bestimmten Voraussetzungen ausgesetzt wird.

9. Verfahren nach zumindest einem der vorhergehenden Ansprüche **dadurch gekennzeichnet, dass** eine Übertragung von medizinischen, patientenspezifischen und/oder therapiebezogenen Daten gegenüber der Übertragung von Datenpaketen der Modulaktualisierungen priorisiert wird, wobei die Priorität der Übertragung am Beatmungsgerät und/oder an der räumlich getrennten Gegenstelle für einzelne Datenarten (z.B. medizinische, patientenspezifische und/oder therapiebezogene Daten; Modulaktualisierungen) individuell einstellbar ist..

10. Verfahren nach zumindest einem der vorhergehenden Ansprüche **dadurch gekennzeichnet, dass** die Übertragung und Durchführung einer Modulaktualisierung unter bestimmten Umständen allen anderen Datenübertragungen vorgezogen wird.

11. Verfahren nach zumindest einem der vorhergehenden Ansprüche **dadurch gekennzeichnet, dass** bei der Verfügbarkeit einer neueren Version der Modulaktualisierung zunächst geprüft wird, ob eine der vorhergehenden Modulaktualisierungen notwendig ist, damit die neue Version der Modulaktualisierung durchgeführt werden kann, wobei dann erst die ältere Version der Modulaktualisierung übertragen und durchgeführt wird, bevor die neuere Version der Modulaktualisierung übertragen und durchgeführt wird.

12. Verfahren nach zumindest einem der vorhergehenden Ansprüche **dadurch gekennzeichnet, dass** bei einer Vielzahl von verfügbaren Modulaktualisierungen für verschiedene Module die Modulaktualisierungen nach einander übertragen und durchgeführt werden und wobei die nächste Modulaktualisierung erst dann übertragen und durchgeführt wird, wenn die vorherige Modulaktualisierung vollständig abgeschlossen ist.

13. Verfahren nach zumindest einem der vorhergehenden Ansprüche **dadurch gekennzeichnet, dass** bei einer Vielzahl von verfügbaren Modulaktualisierungen für verschiedene Module die Modulaktualisierungen gleichzeitig übertragen und installiert werden.

14. Verfahren nach zumindest einem der vorhergehenden Ansprüche **dadurch gekennzeichnet, dass** die Übertragung der Modulaktualisierung basierend auf der Verbindung zwischen dem Beatmungsgerät und der räumlich getrennten Gegenstelle automatisch durch das Beatmungsgerät unterbrochen wird.

15. Verfahren nach zumindest einem der vorhergehenden Ansprüche **dadurch gekennzeichnet, dass** eine Übertragung der Modulaktualisierung bei einer Verbindung zwischen dem Beatmungsgerät und der räumlich getrennten Gegenstelle über eine Mobilfunkverbindung (LTE, 3G, etc.) nicht stattfindet und unterbrochen wird, falls die Verbindung zwischen dem Beatmungsgerät und der räumlich getrennten Gegenstelle auf eine solche Mobilfunkverbindung wechselt.

16. System zur Beatmung umfassend zumindest ein Beatmungsgerät (1) und eine räumlich getrennte Gegenstelle (10), **dadurch gekennzeichnet, dass** die Firmware und die Software des Beatmungsgerätes (1) in einzelne Module (20, 30, 40, 50, 60, 70, 80, 90) unterteilt ist und zu jedem Modul (20, 30, 40, 50, 60, 70, 80, 90) separate Aktualisierungen in Form von Modulaktualisierungen bereitgestellt werden, diese Modulaktualisierungen von der Gegenstelle (10) über zumindest eine Schnittstelle des Beatmungsgerätes (1) auf das Beatmungsgerät (1) übertragen werden und die Modulaktualisierungen auf dem Beatmungsgerät (1) durchgeführt werden.
